# EUROPEAN PATENT SPECIFICATION

(11) **EP 0 293 447 B1**
(45) Date of publication and mention of the grant of the patent: **31.08.1994**
(21) Application number: 88900311.7
(22) Date of filing: 01.12.1987
(51) Int. Cl.: G01N 33/543, G01N 33/558

(54) **A DEVICE AND METHOD FOR SELF CONTAINED SOLID PHASE IMMUNODIFFUSION ASSAY**
VORRICHTUNG UND VERFAHREN ZUR FESTPHASEN-IMMUNODIFFUSIONS-UNTERSUCHUNG
DISPOSITIF ET PROCEDE D'ANALYSE D'IMMUNO-DIFFUSION A PHASE SOLIDE ISOLEE

(30) Priority: 03.12.1986 US 938003
(43) Date of publication of application: 07.12.1988
(73) Proprietor: NEW HORIZONS DIAGNOSTICS CORPORATION, Columbia, MD 21045 (US)
(72) Inventor: BERNSTEIN, David, Skyesville, MD 21784 (US)
(74) Representative: Patentanwälte Bartels und Partner
(86) International application number: PCT/US87/03169
(87) International publication number: WO 88/04431

(56) References cited:
- US-A- 3 960 491
- US-A- 4 184 483
- US-A- 4 196 167
- US-A- 4 235 601
- US-A- 4 353 868
- US-A- 4 409 988
- US-A- 4 613 567
- US-A- 4 673 657
- US-A- 4 703 017
- ABSTRACTS OF THE ANNUAL MEETING OF THE AMERICAN SOCIETY FOR MICROBIOLOGY,Washington, D.C., 1986, abstract no. C-266; D. BERNSTEIN et al.: "Rapidimmunodiffusion enzymatic assay (RIDE) for group A Streptococci"

## Description

### BACKGROUND OF THE INVENTION

In order to determine the condition of a patient, and to minimize the diseased state, the need for a rapid diagnosis and appropriate treatment by health care professionals is apparent. Diagnosis of many conditions can be facilitated through the determination or quantitation of antibodies, antigens, nucleotide fragments, and other analytes from a biological specimen, which are indicative of a particular disease state or condition. A rapid, sensitive, specific, and simplistic assay is extremely useful for emergency situations, field testing, physicians offices and in home diagnostics. As diagnostic tests become more simple and easier to perform, they are being performed away from the professional clinical laboratory setting to physicians offices and even to the home, where untrained or poorly trained individuals perform the tests usually following product insert instructions alone. These assays are useful provided they are performed properly and are safe to handle for the user. Assays that require multiple steps, have multiple reagents, and have limited storage conditions are prone to misuse, especially if they are performed by individuals without adequate training or skills.

Many types of ligand receptor assays have been developed and commercialized. These assays are less expensive if capital equipment can be eliminated, such as scintillation counters, fluorometers, and colorimeters in the case of radioimmunoassay, fluorescent immunoassay, and enzyme immunoassay respectively. Non instrumental assays, such as latex agglutination, enzyme immunoassays on strips, tubes, membrane or filters have increased the usefulness and ease of performance of immunodiagnostic testing, but are still cumbersome requiring washing steps, multiple reagent additions and usually refrigerated storage conditions.

In some assays amplification or growth of viruses and bacteria are desirable before testing to increase the sensitivity of detection. In other assays adsorption steps to remove interferring substances or inhibitors of the ligand receptor assay, or long incubation of reagents are necessary to perform an assay. Each step for an assay increases the difficulty of testing for the minimally trained individual and any device that would reduce user error would improve diagnostic testing.

In USA 4 184 483 a device is disclosed for keeping an unaerobic microbial sample moist and viable for a period of time after it is collected. This is accomplished through the use of a swab with which the culture is collected and a tube which receives the culture carrying swab and bathes the swab in the culture sustaining liquid. A barrier member is provided which extends across the tube and has an opening through which the swab passes forming a substantially leak proof barrier around the shaft of the swab. Thus the culture carrying swab is kept in a leak proof chamber which extends from the bottom of the tube to the barrier member.

Horrisberger et al (J. Histo Cytochem volume 25: 295-305, 1977) described the use of colloidal gold particles in an immunoassay. Leuvering in US patent number 4,313,734 also describe such an immunoassay. Cerny in PCT/US patent application number 85/02534 describes a solid phase diffusion assay using gold sol particles as an immunolabel which can be visualized by the naked eye on a capture membrane, and requires no washing step. Bernstein et al ABSTRACTS OF THE ANNUAL MEETING OF THE AMERICAN SOCIETY OF MICROBIOLOGY (1986), abstract number C-266; "Rapid immunodiffusion enzymatic assay (RIDE) for group A streptococci presented and described a rapid immunodiffusion enzyme labeled antibody assay for Group A streptococci on a membrane in which there is no washing step. Within this multiple step procedure a neutralized micronitrous acid extract of a throat swab culture is physically squeezed from the swab and the resultant expressed liquid is mixed with enzyme labeled antibody, which in turn is collected into a capillary tube. The capillary tube is then used to transfer the reactants to a capture membrane. Afterwards the membrane is placed in a substrate solution and examined for color reaction. Gould and Zuk in US patent number 4,552,839 describe the use of colored or dyed beads in a solid phase immunoassay. Through the introduction of colored immunolabelled binding reagents (i.e. gold sol particles, dyed particles, dye encapsulated liposomes, etc.) and the removal of washing steps it becomes possible to perform receptor ligand assays in a closed system with the sequential additions of all reagents within that system.

A number of antigens of interest in the diagnosis of infectious disease are collected with a sterile swab on a shaft to remove the organisms from the suspected infected area or test site (wounds, lesions, blood, tissues, pus, fluids, etc.). The swab is generally used to transfer organisms to a suitable media for culturing which may take as long as 48 hours for growth of bacteria, and 2 weeks for viruses. If the organisms are viable and do grow, then their identification could be made by biochemical, morphological or immunological methods. This time consuming method is slowly becoming replaced by more rapid immunological testing methods or DNA probe methodologies.

In many immunoassays that utilize a swab for collection of antigens or cells, the swab is placed in a solution to release the antigenic materials or cells after collection. It may be necessary to use enzymes, acids, detergents, etc. to solubilize or breakdown the antigens to expose antigenic determinants. The extracted material can then be used in an immunoassay by removing the fluid from the swab and mixing it with other reagents or adding the other reagents directly to the swab extract. In the case where membrane or filters are used to capture the immunoreactants, it is necessary to bring the fluid containing the immunoreactants in contact with the filter or membrane.

In addition, where extraneous cells or debris may interfere with an assay, it may be necessary to have a prefilter (larger pore size filter or membrane) present between the swab and the capture membrane or capture filter to retain these unwanted components.

In some assays, where antigen expression may be low, amplification can be achieved if the organisms are first cultured and then tested. If the culturing and the testing could be performed in a single device, then testing would be simplified. In some assays where there are inhibitors, cross reactive products, or clotting factors, red blood cells, etc., it may be necessary to add adsorbant materials (i.e. beads, kaolin, antibody coated particles, antigen coated particles, or lectin coated particles), anticoagulants, or buffers etc. before the ligand receptor assay can be performed.

### BRIEF DESCRIPTION OF THE INVENTION

It is an object of the present invention, in one preferred form, to provide a novel test device that utilizes a swab or swab-like material (a shaft with a porous or fibrous absorbant material at one end) to collect a sample and to be able to react the swab with all the necessary reagents which are included within the device, and then to use the swab to transfer the reactants sequentially to other reactants if necessary, and finally to a reaction zone where the specific labelled reactant can be captured and visualized.

It is another object of the present invention, in a second preferred form, to provide a prefabricated assay device in the form of a laminate. This laminate in cooperation with a swab or other sample collector device provides a means for performing many types of ligand receptor assays. The laminate can be used in many different assay formats.

It is a further object of the present invention to provide a test device useful in performing ligand receptor assays to detect antigens, haptens, antibodies, DNA or RNA fragments, wherein the user is not required to dispense any of the reagents.

It is a particular object of the present invention to provide a test device that can be stored at nonrefrigerated temperatures, and can be utilized to perform an assay on a biological specimen or fluid without any additional reagents having to be provided to the test device.

In addition it is a further object of the present invention to provide a test device which can utilize lyophilized reagents that can be reconstituted in situ within the device.

The present invention maximizes the safety and ease of performance of ligand receptor assays through the use of apparatus designed to enable a biological specimen to be obtained by a collection device comprising a shaft and an attached tip of adsorbent or absorbent porous or fibrous material (i.e. rayon, dacron, cotton swab). In the first form of the invention, the tip is inserted into a cylindrical tube. The cylindrical tube contains a sealed vessel, or chamber, or plurality of sealed vessels or chambers in sequential order. The seal will break away or collapse when pressure of the collection device (swab) is exerted on the seal by physically pushing the collection device into and through each vessel. These sealed vessels may contain media, extraction reagents, diluents, labelled antibodies, labelled antigens, labelled lectins, anticoagulants, adsorbants, inactivators, etc. which mix with the biological specimen collected on the collection device. The reagents in these vessels may be lyophilized, enabling long term storage at non refrigerated temperature. The vessels are fixed in position in the cylindrical tube to enable the seals to be broken when physical pressure is exerted on the shaft of the collection device. The collection device holder has appropriate stop points to allow for the collection device tip to enter the appropirate vessel and mix with its contents. A key feature of the vessels are that the tip and shaft of the collection device can pass through each of the vessels into a lower portion of the cylindrical tube and attached lower portion comprising a ligand receptor reaction area. This ligand receptor area is part of a pre-fabricated laminated assay device.

The ligand receptor area is comprised of a capture'membrane or a filter that will allow unbound reactants to pass through by diffusion and retain the appropriate labelled members of the binding pair. The capturing membrane or filter may be coated with a member of the binding pair to capture the reactants. If capture particles are used, then the capture filter is utilized to retain the particles and allow unbound free labelled antigen or antibody to diffuse through. A prefilter may be used between the collector tip and the capture or filter to remove any nonspecific binding due to debris. An additional absorbant material can be placed behind the capture membane to increase the uptake of fluid. In either case a specific volume of reactants can be absorbed by controlling the size of the filter and absorbant materials. The configuration of the lower portion allows the collection device to come into physical contact with the prefilter, capture membrane or capture filter. The pre-fabricated assay device is comprised of sequential layers of at least one porous membrane, an impervious shield having at least one opening, and a filter means.

### DESCRIPTION OF THE DRAWINGS

Fig. 1 is a cross-sectional view of a first form of the invention showing the collection device holder, the collection device, the tube, the sealed reagent compartments and the lower ligand receptor transfer area;
Fig. 2 is a perspective view of the basic structure of the collection device holder and collection device of Fig. 1 including the grooves for guiding the movement of the collection device through the apparatus;
Fig. 3 is a perspective view of the basic structure of the tube of Fig. 1, its compartmentalized reagents, and the nodule which fits into the groove of the collection device holder;
Fig. 4 is a perspective view of the sealed compartments (i.e. vessels) of the apparatus of Fig 1;
Fig. 5 is a cross-sectional side view of the lower portion of the apparatus of Fig. 1 showing the final position of the collection device tip at the window of the ligand receptor area;
Fig. 6 is an exploded perspective view of the ligand receptor test area as used in the apparatus in Fig 1;
Fig. 7 is an exploded perspective view of the pre-fabricated assay device according to a second form of the invention;
Fig. 8 is a pictoral view of the sample collection device in contact with the pre-fabricated assay device using the second form of the invention;
Fig. 9 is a perspective view of a prefabricated assay device and sample collection device being sandwiched between an upper hydrophobic pressure plate 29 and a lower flat hydrophobic plate 30 according to a third form of the invention; and
Fig. 10 is a perspective view of a prefabricated assay device showing a modification of the third form of the invention having a sample collection device positioned on a lower flat hydrophobic plate 30 which is hinged to a hydrophobic upper pressure plate 29.

### DESCRIPTION OF THE PREFERRED EMBODIMENTS

In the following description, the various forms of the apparatus and the method of the invention will be described in exemplary terms only, for an antigen determining an immunoassay test. This discussion, however, is simply to illustrate the structure and use of the apparatus and the technique and steps of the method. The various forms of the apparatus clearly can be used for any ligand receptor assay in which washing steps have been eliminated and transfer of the reactants to or through a porous membrane or filter is used. The best modes, as described hereinafter, are accordingly, to be considered exemplary and not limiting as to the scope and concept of the invention.

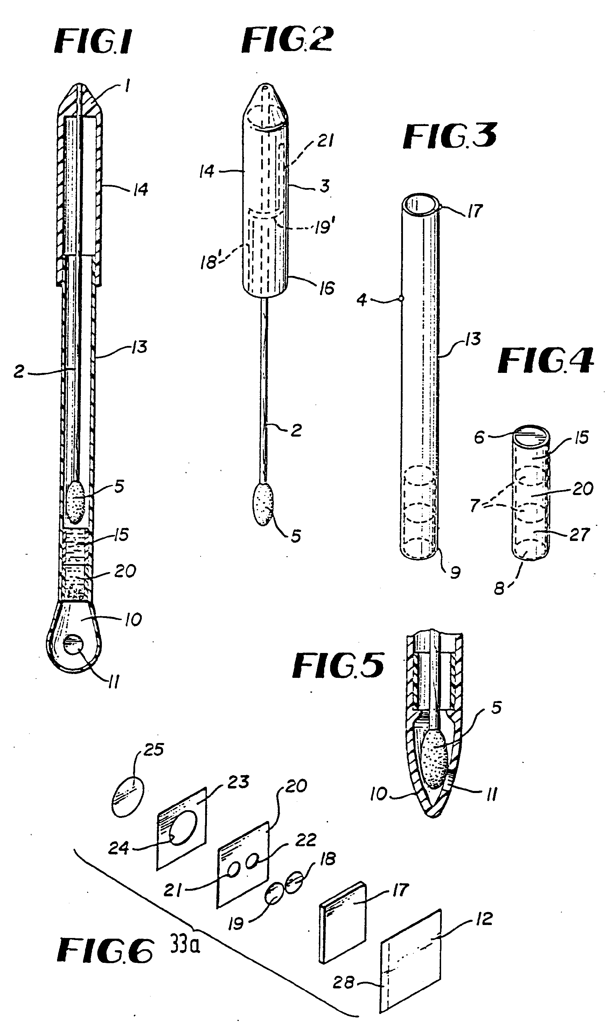

Referring first to Fig. 1 for a general depiction of the apparatus, the inventive apparatus comprises a collection device holder 14 which is comprised of a restrictive portion 1 that hold the shaft of the collection device 2 in place, a cylindrical tube 13 which is comprised of one or more sealed reagent compartments 15 and 20, and a lower ligand receptor reaction area 10 with a hole of window 11. Area 10 is drawn enlarged, but can be smaller, see Fig. 5 for example.

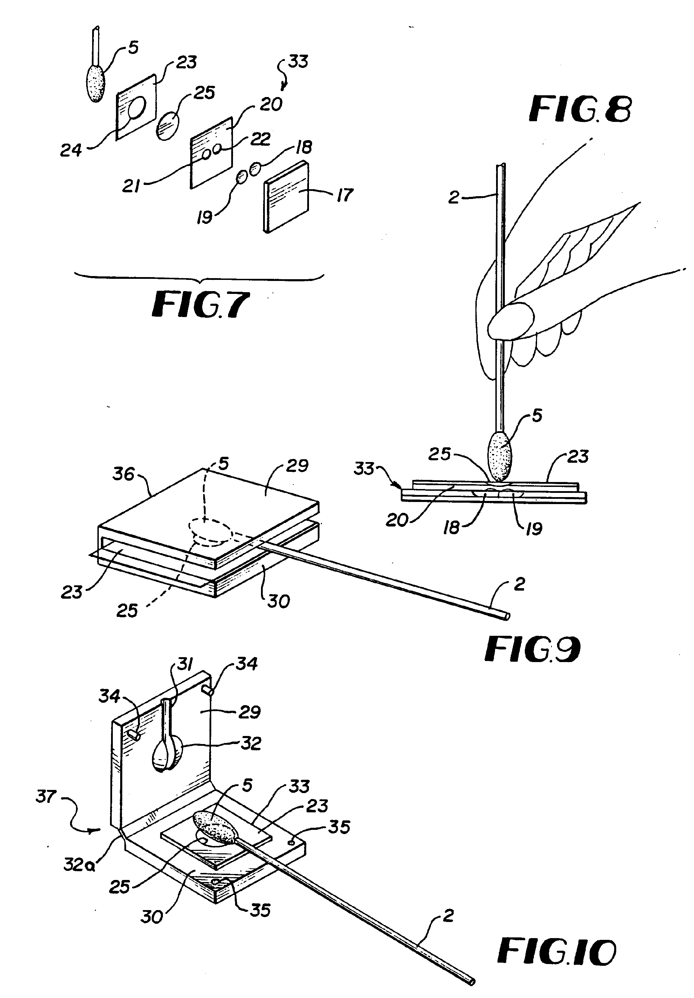

Referring to Fig. 2, the collection device holder 14 has a nodule 16 which positions onto the cylindrical tube and prevents the apparatus from being accidentally opened. Nodule 16 can be heat sealed or made to cooperate with a groove formed in tube 13, or the cooperating nodule 17 shown in Fig. 3. or other means can be provided to accomplish the sealing function. When a sample is to be taken, the collection device holder is removed and separated from the cylindrical tube by twisting and pulling up on the collection device holder. This frees up the collection device holder which is then used to collect the test sample (i.e. throat swab, pus, blood, urethral swab, etc.) by allowing the collection device tip 5 to come into contact with the suspect tissue, fluid, wound, etc.

Referring to Fig. 2 and Fig. 3, after obtaining a test sample, the collection device holder is replaced onto the cylindrical tube 13 and turned until the nodule 4 (Fig. 3) on the cylindrical tube is in alignment with the groove 18′. The collection device holder is then manually forced downward until the nodule 4 stops at the horizontal groove 19′. When the nodule 4 is in contact with horizontal groove 19′, then simultaneously the tip 5 will have broken through the first seal (Fig. 4), mixing with the contents of the first chamber 15, then breaking through seal 7 and emptying its contents into chamber 20. The number of independent chambers is related to the number of required reagent additions and incubation steps. One chamber or a plurality of chambers could be used and the mixing of reagents controlled using the principles of nodule 4 and grooves 18′, 19′ as previously described. In the preferred embodidment, the collection device holder is turned to the right and then back and forth using groove 19′ to mix the contents of vessel 20 through the simultaneous turning of the collection device tip.

Referring to Figs. 2 and 3, after an appropriate incubation time, the collection device holder 14 is turned to the right and thus aligning nodule 4 (Fig. 3) with groove 3 (Fig. 2) and then manually forced downward until the movement of nodule 4 is stopped by the groove end 21 (Fig. 2).

Referring to Figs. - 5 and 6 the lower portion 10 may be physically one piece with the cylindrical tube 13 or may be an attached separate piece. When the nodule 4 is in contact with the groove end 21, then the collection device tip 5 is in contact with the prefilter membrane 25 through the window 11. The reactants flow through the prefilter membrane through holes 24 of impervious shield 23 which holds the prefilter membrane 25 against hole or window 11. The shape of the lower portion 10 is configured to enhance contact of the collection device tip 5 with the prefilter or reaction membranes. If preferred, the prefilter could be placed on the inside wall of the window 11. In any case, the reactants flow through holes 21 and 22 of shield 20 which holds membranes 18 and 19 respectively in place. The holes 21 and 22 restrict the flow of the reactants through a capture membrane 19 and a control membrane 18 and enhance the signal of the reaction by concentrating the labelled ligand or receptor binding pairs into a small area. Absorbant 17 absorbs excess fluid diffusing through the membranes. When an appropriate volume of fluid has diffused through the membranes, usually by saturation of the absorbant, the capture and control membrane are examined within the holes 21 and 22 respectively by lifting the tab 28 of the adhesive tape 12.

In the preferred form of the invention, the assay results are examined by the naked eye since color changes are the result. However the invention is not limited in this manner and the "examination" of test results could be done with instruments or otherwise dependent upon the protocol of the particular assay.

Adhesive tape 12 holds the absorbant 17 in place and applies the necessary pressure to insure diffusion of fluid through the various layers of the ligand receptor test device 33A. The color intensity of the capture membrane 18 is compared to the color intensity of the control membrane 19. A positive result is determined by viewing a more intense color in the capture membrane than in the control membrane. A negative result is determined by no significant color or the same weak color in the capture and control membranes. In competitive inhibition assays the positive and negative results are reversed. In the performance of drug analyte assays, the size of the ring of color in a single larger capture membrane is related to the concentration of drug in the test sample. The design of the ligand receptor area, the coating of reagents on the membranes, and the addition or deletion of capture or control membranes are dependent on the particular type of assay being performed. The capture membrane can be coated with antigen or antibody, or other complementary ligands or receptors and can be used to determine the presence of different antigens or antibodies. The number of chambers used in the apparatus is dependent upon the type of assay and can contain diluents, media for growth amplification of microorganisms, lyophilized labelled ligands or receptors, etc. The seal 7 (Fig. 4) may be attached to two chambers simultaneously or may be independent. Therefore the chambers could be attached to each other or be independent.

Referring to Fig. 6, the prefilter 25 is fixed around the periphery of hole 11 in tube 10 by taping, gluing, heat sealing, chemically sealing, sonic welding, or the like. Impervious shield 23 is a single side adhesive coated laminate having adhesive on the side facing the prefilter 25. The impervious shield laminate 23 with hole 24 fixes and exposes the prefilter to the tube 10 and hole 11. The impervious shield 20 can also be a single side adhesive coated laminate which fixes membranes 18 and 19 onto absorbent 17. Absorbent 17 is fixed on the side opposite to the positioned membranes with adhesive tape 12. The adhesive tape 12 fixes the assay device 33A to tube 10. Alternative means of fixing can include clamps, glues, or the like. In order to examine a test result on the membranes 18 and 19 it is necessary to separate the impervious shields 23 and 20 from each other to expose the membranes. By pulling back adhesive tape 12, a portion of the assay device 33A consisting of shield 20, membranes 18 and 19, and absorbent 17 separates from filter 25 and shield 23, thereby exposing membranes 18 and 19 for examination.

Referring to Fig. 7, the pre-fabricated assay device 33 is a laminate comprised of a prefilter membrane 25 held in place by a hydrophobic impervious shield 23 having a hole 24 through which reactants from absorbent tip 5 can flow into the prefilter. When absorbent tip 5 is pressed into contact with prefilter 25, the flexible prefilter is forced into contact through holes selectively clustered and sized 21 and 22 of impervious shield 20 with control and capture membranes 18 and 19. The fluid reactants from tip 5 flow from prefilter 25 to both membranes 18 and 19 simultaneously. Absorbent 17 is in contact with membranes 18 and 19 and the fluid reactants diffuse through membranes 18 and 19 to absorbent 17. At the conclusion of a ligand receptor assay the impervious shield 23 with attached prefilter 25 is removed and membranes 18 and 19 are examined by the naked eye, or otherwise, for any color change, or the like.

Fig. 8 is a perspective view of a collection device tip 5 being manually brought into contact with an assembled prefabricated assay device 33. The fluid of a labelled ligand or receptor reagent in combination with sample is transferred from tip 5 through prefilter membrane 25 and through control and capture membranes 18 and 19 to absorbent 17. This shows the general utility of the invention devices 33 and 33A to be used in many different environments.

Fig. 9 is a perspective view of a sandwich device 36 for holding collection device tip 5 under pressure against a prefabricated assay device 33. Hydrophobic upper pressure plate 29 is movable and hinged to hydrophobic lower flat plate 30. After reacting sample collection device tip 5 with labelled ligand or labelled receptor by manipulating shaft 2, the sample collection device tip 5 is positioned horizontally in contact with prefilter membrane 25 and pressure is applied to the device forcing the absorbent tip 5 to push the prefilter 25 into contact with porous membranes 18 and 19 and allowing the reactants to flow through and into the pre-fabricated assay device 33. Pressure can be applied to pressure plate 29 by means of a weight, clamp, manually squeezing, or the like. After an appropriate period of time to allow the fluid reactants to penetrate the assay device 33, pressure is removed from the hinged sandwich device and the impervious shield 23 is lifted to allow the control and capture membranes of the pre-fabricated assay device to be examined.

Fig. 10 is a perspective view of a hinged sandwich device 37 for applying pressure to sample collection device tip 5 and the pre-fabricated assay device 33. Upper hydrophobic pressure plate 29 is contoured as at 31 to the sample collection device 2,and 5 to maximize the amount of fluid that can flow into the prefabricated assay device 33 which is positioned on the lower hydrophobic plate 30. After the sample collection device has reacted with labelled ligand or labelled receptor the sample collection device is manually placed within the hinged sandwich device 37 with sample collection device tip 5 in contact with prefilter membrane 25 and pressure is applied to and maintained by the hinged sandwich device 33. The hinged sandwich device 33 consists of a hinge 32 and a clamping mechanism such as protrusions 34 from upper pressure plate 29 and a complementary fitted piece such as the orifices 35 which will produce a friction fit and maintain the closed clamped position of the hinged sandwich device 33. After an appropriate time period the hinged sandwich device is opened and the prefilter membrane 25 is removed to expose the control and capture membranes of the prefabricated assay device 33.

In Figs. 9 and 10, the assay device 33 can be layed loosely, or affixed as by glueing or the like to one of the plates 29 or 30. Also a form fitted depression to hold the device 33 could be formed in a plate 29 or 30.

### DEFINITIONS

In the following section certain terms used in the specification and claims herein shall be understood to have the following meanings:
- Ligand:: Any organic compound for which a receptor naturally exists or can be prepared including haptens, antigens, sugars, vitamins, peptides and the like.
- Receptor:: Any compound capable of recognizing a specific molecular configuration of a ligand including antibodies, lectins, enzymes, nucleic acids, Fab fragments, avidin and the like.
- Ligand receptor reaction:: Any binding between a receptor and its complementary ligand.
- Porous membrane:: any porous solid matrix including porous spreading layers, bibulous papers, filters, and the like.
- Coated porous membrane:: A porous membrane that has a ligand or receptor covalently or noncovalently attached to its surface.
- Impervious shield:: any hydrophobic material that will not allow diffusion of fluid to occur through its surface. This material can be plastic, a plastic adhesive laminate, and the like.
- Chamber:: a partitioned space, vessel, reservoir, compartment or the like.

### The following example is illustrative:

### EXAMPLE 1

A RAPID IMMUNODIAGNOSTIC TEST FOR GROUP A STREPTOCOCCI. Group C phage associated lysin enzyme which is effective in fragmenting and solubilizing the Group A streptococcal polysaccharide was diluted in a buffer of .05M citrate phosphate pH 6.1 containing 0.005 EDTA, 0.005 DTT, 0.1% rabbit IgG, 0.05% sodium azide and mixed with rabbit anti streptococcal Group A coated gold sel particles (OD⁵¹⁸ 1.5) diluted in a buffer of 0.02 Tris pH 8.2 containing 1.0% BSA, 0.2% sodium heparin, 0.5% n-acetylglucosamine and 0.02% sodium azide in a ratio of 3 parts lysin reagent to 1 part antibody gold sol reagent. The combined reagent was sterile filtered through a 0.2 micron cellulose acetate filter and 200 microliters were aliquoted into acrylic walled reaction cup vessels, having an aluminum foil sealed bottom. The aliquots were frozen and lyophilized. The reaction cup vessels were sealed with aluminum foil and contact cement under nitrogen. Another reaction vessel was cemented to the aluminum foil lid of the first vessel. Two hundred microliters of distilled water was added to the second vessel and then cemented and sealed with aluminum foil. The vessels were placed and positioned into the cylindrical tube. The ligand receptor area was prepared by coating nitrocellulose membranes with rabbit anti Group A streptococcal antibody for the capture membranes, and normal rabbit immunoglobulin for the control membrane. The membranes were dried and fixed to a diacetate laminate which had 1.5mm diameter holes for each membrane. A 1.2 micron cellulose acetate prefilter was used to cover the hole (window) in the lower portion of the tube. A dacron tipped swab was seeded with varying concentrations of group A streptococci. The swab was placed into the cylindrical tube and forced downward to break the first two seals on the reaction vessels. The swab was incubated for 4 minutes at room temperature allowing the lysin enzyme to solubilize the Group A streptococcal polysaccharide and the reaction of the gold labelled anti Group A antibody to form complexes with the released polysaccharide. After the four minutes, the swab was forced downward through the third seal into the lower portion, coming in contact with the ligand receptor area 33. The fluid diffused through the prefilter into the capture and control membranes. After 30 seconds the tab 28 on the ligand receptor area 33 was pulled away from the lower portion and examined by eye. A distinct color reaction with 2 x 10³ organisms of Group A streptococci could be distinguished in the capture membrane compared to the colorless control membrane.

## Claims

1. A self-contained ligand receptor device, the combination comprising;
a) a collection device having an elongated shaft (2) with an absorbent tip (5) for collecting a specimen;
b) a tube (13), cooperative with said collection device, said tube (13) having an interior, an open end and a distal end:
c) at least one sealed chamber (15, 20, 27) in said tube (13);
d) a number of reagents equal to the number of said at least one chamber (15, 20, 27) located one reagent in each said at least one chamber (15, 20, 27);
e) means to seal each said reagent in a respective one of said at least one chamber (15, 20, 27);
f) said seal means comprising frangible seals (7);
g) ligand receptor reaction means including at least one porous membrane (18, 19);
h) a hole (11) formed in said tube (13) in predetermined spaced relation to said distal end thereof;
i) means (20) to fix said ligand receptor reaction means to said tube (13) covering said hole (11) with said least one porous membrane (18, 19) exposed to said interior of said tube (13); and
j) the length of said shaft (2) being such as to permit said absorbent tip (5) to reach through all of said at least one chamber (15, 20, 27) to mix all of said at least one reagent and to permit fluid diffusion between said tip (5) and said at least one membrane (18, 19); whereby
k) a labelled ligand or a labelled receptor is immobilized on said ligand receptor reaction means (33a) and the result of said assay can be examined.

2. The device of claim 1, a guiding mechanism for said collection device, said guiding mechanism comprising guide and stop means (3, 18′, 19′), nodule means (4) on said tube (13), and said guide and stop means (3, 18′, 19′) and said nodule means (4) cooperating with each other causing sequential breaking of said at least one frangible seal (7) and moving said absorbent tip (5) completely through at least one chamber (15, 20, 27) when said absorbent tip (5) passes through all of said at least one chamber (15, 20, 27).

3. The device of claim 1, wherein said at least one reagent comprises a labelled ligand or a labelled receptor.

4. The device of claim 3, further comprising at least one other chamber (15, 20, 27), and microbiological growth media in said other chamber (15, 20, 27).

5. The device of claim 3, further comprising at least one other chamber (15, 20, 27), and an extraction reagent for exposing antigenic determinants or hidden epitopes of an antigen in said other chamber (15, 20, 27).

6. The device of claim 3, wherein said labelled ligand or labelled receptor reagent is lyophilized.

7. The device of claim 3, wherein said labelled ligand or labelled receptor reagent comprises a chromophore.

8. The device of claim 7, wherein said chromophore is selected from the group consisting of dyes, dyed particles, pigments, metal sol particles, or dye encapsulated liposomes.

9. The device of claim 8, wherein said chromophore is a metal sol particle selected from the group consisting of gold, silver, or the combination of gold and silver.

10. The device of claim 1, wherein said absorbent tip (5) for collecting a specimen is sterile.

11. The device of claim 3, further comprising at least one chamber (15, 20, 27), and adsorption particles in said other chamber (15, 20, 27), said adsorption particles comprising binders to remove inhibiting or cross reactive substances from said device; and at least one other porous membrane (25), and
a) said adsorption particles are being larger than said labelled ligands or labelled receptors, and
b) said at least one other porous membrane (25) having an effective pore size smaller than said adsorption particles and larger than said labelled ligands or labelled receptors.

12. The device of claim 1, wherein said at least one porous membrane (18, 19) is a plurality of porous membranes (18, 19) at least one membrane (19) of which is coated with different ligands or receptors.

13. The device of claim 3, wherein one of said at least one chamber (15, 20, 27) contains ligand or receptor coated particles which are larger in mean particle size diameter than said labelled ligand or labelled receptor and the effective pore size of said at least one porous membrane (18, 19).

14. A method of performing a ligand receptor assay using sample collector means, comprising the steps of;
a) putting a specimen on said sample collector means;
b) contacting said specimen on said sample collector means with at least one labelled reactant which is required to perform said assay;
c) providing at least one porous membrane (18, 19) as required to perform said assay;
d) causing liquid diffusion from the reactants on said sample collector means after said step b) to all of said at least one porous membrane (18, 19) simultaneously; and
e) examining all of said at least one porous membrane (18, 19) after performance of all of steps a) to d) above; whereby any color change or the like caused by a labelled ligand or a labelled receptor being captured or not being captured on said at least one membrane (18, 19) in accordance with the protocol of said assay can be readily observed.

15. The method of claim 14, and the additional steps of providing two of said porous membranes (18, 19), causing one of said two porous membranes (18, 19) to be a control membrane (18) and the other of said two porous membranes (18, 19) to be a capture membrane (19) and, and positioning said two membranes (18, 19) so that said two membranes (18, 19) can be observed simultaneously.

16. The method of claim 14, and the additional step of filtering said reactants during said step d) using filter means (25) having a pore size selected to be large enough to permit said liquid diffusion of said step d) and small enough to prevent debris larger than said selected pore size and the like which may be on said sample collector means from contacting any of said at least one porous membrane (18, 19).

17. The method of claim 14, and performing said step d) including the sub-step of creating physical pressure at least indirectly between said sample collector means and all of said at least one porous membrane (18, 19) simultaneously.

18. The method of claim 14, wherein said steps c) and d) are performed using a pre-fabricated assay device (33), and the steps of constructing said assay device (33) in the form of a laminate comprising sequential layers as follows:
i) said at least one porous membrane (18, 19);
ii) impervious shield means (20), and the steps of providing at least one opening (21, 22) in said impervious shield means (20) equal in number to the number of said at least one porous membrane (18, 19), and sizing and clustering said at least opening (21, 22) so that said step d) can be performed through all of said at least one opening (21, 22) simultaneously; and
iii) filter means (25), and selecting a pore size for said filter means (25) large enough to permit liquid diffusion of said step d) and small enough to prevent debris larger than said selected pore size and the like which may be on the sample collector means from contacting any of said at least one porous membrane (18, 19).

19. The method of claim 18, and the additional steps performed during said construction of said laminated device step of providing an absorbent means layer (17), and locating said absorbent means layer (17) next to said at least one porous membrane (18, 19) on the side thereof opposite said impervious shield means (20).

20. A pre-fabricated assay device (33) for use with a sample collection device comprising sequential layers of:
a) at least one porous membrane (18, 19);
b)
i) impervious shield means (20) formed with at least one opening (21, 22) equal in number to the number of said at least one porous membrane (18, 19);
ii) means for sizing and clustering said at least one opening (21, 22) so that liquid diffusion between said at least one porous membrane (18, 19) and a reactant brought into contact with said assay device (33) can occur through all of said at least one opening (21, 22) simultaneously; and
c) filter means (25), and said filter means (25) having a pore size large enough to permit said liquid diffusion and small enough to prevent debris larger than said selected pore size from contacting any of said at least one porous membrane (18, 19) and; said pre-fabricated assay device (33) and said sample collection device together comprising a self-contained ligand receptor assay.

21. The device of claim 20, and a sample collection device comprising an elongated shaft (2) and an absorbent tip (5).

22. The device of claim 21, and a container comprised of liquid labelled receptor or labelled ligand reagent.

23. The device of claim 22, wherein the said labelled reagent comprises a chromophore.

24. The device of claim 23, wherein the said chromophore is selected from the group consisting of dyes, dyed particles, pigments, metal sol particles, or dye encapsulated liposomes.

25. The device of claim 21, and a sandwich device (37), said sandwich device (37) comprising a pair of plate means (29, 30), means (32a) to hinge said plate means (29, 30) together along one respective side of said pair of plate means (29, 30), positioning said pre-fabricated assay device (33) between said plate means (29, 30), and means (34) to apply pressure to said tip (5) when said tip (5) is in contact with said pre-fabricated assay device (33).

26. The device of claim 20, and said assay device (33) including an absorbent layer (17) in contact with said at least one porous membrane (18, 19) on the side thereof opposite said impervious shield (20).

27. The device of claim 25, and said assay device (33) including an absorbent layer (17) in contact with said at least one porous membrane (18, 19) on the side thereof opposite said impervious shield (20).

28. The device of claim 25, said means to apply pressure comprising clamping means (34).

29. The device according to one of the claims 25 to 28, said sandwich device means to apply pressure comprising contoured protrusion means (31, 32) formed on said sandwich device (37).

## Patentansprüche

1. In sich geschlossene Ligand-Rezeptor-Vorrichtung, wobei die Kombination umfaßt;
a) eine Sammelvorrichtung bestehend aus einem verlängerten Stäbchen (2) mit einer absorbierenden Spitze (5) zum Sammeln einer Probe;
b) ein Röhrchen (13), das mit der genannten Sammelvorrichtung zusammenwirkt und ein Inneres, ein offenes Ende und ein distales Ende aufweist;
c) wenigstens eine abgedichtete Kammer (15, 20, 27) in dem genannten Röhrchen (13);
d) eine Anzahl von Reagenzien entsprechend der Anzahl der genannten wenigstens einen Kammer (15, 20, 27) so daß in jeder der genannten wenigstens einen Kammer (15, 20, 27) ein Reagenz untergebracht ist;
e) Mittel zum Abdichten jedes genannten Reagenzes in einer jeweiligen der genannten wenigstens einen Kammer (15, 20, 27);
f) wobei das genannte Abdichtungsmittel zerbrechliche Abdichtungen (7) umfaßt;
g) Ligand-Rezeptor-Reaktionsmittel, welches wenigstens eine poröse Membran (18, 19) einschließt;
h) ein Loch (11), das in dem genannten Röhrchen (13) in vorbestimmter räumlicher Beziehung zu dem genannten distalen Röhrchenende gebildet ist;
i) Mittel (20), um das genannte Ligand-Rezeptor-Reaktionsmittel an das genannte Röhrchen (13) zu befestigen, wobei das genannte Loch (11) mit der genannten wenigstens einen porösen Membran (18, 19) belegt wird, die gegenüber dem genannten Inneren des genannten Röhrchens (13) freiliegt; und
j) wobei die Länge des genannten Stäbchens (2) derartig bemessen ist, daß die genannte absorbierende Spitze (5) durch alle der genannten wenigstens einen Kammer (15, 20, 27) reichen kann, um alle der genannten wenigstens einen Reagenzien zu mischen und um die Diffusion der Flüssigkeit zwischen der genannten Spitze (5) und der genannten wenigstens einen Membran (18, 19) zu erlauben; wodurch
k) ein markierter Ligand oder ein markierter Rezeptor auf dem genannten Ligand-Rezeptor-Reaktionsmittel (33a) festgehalten wird und das Ergebnis der genannten Vorrichtung untersucht werden kann.

2. Vorrichtung nach Anspruch 1 mit einem Führungsmechanismus für die genannte Sammelvorrichtung, wobei der genannte Führungsmechanismus Führungs- und Haltemittel (3, 18′, 19′) und Aussparungen (4) an dem genannten Röhrchen (13) enthält und wobei die genannten Führungs- und Haltemittel (3, 18′, 19′) und die genannte Aussparung (4) miteinander zusammenwirken, wodurch ein aufeinanderfolgendes Aufbrechen der genannten wenigstens einen zerbrechlichen Abdichtung (7) und eine vollständige Bewegung der genannten absorbierenden Spitze (5) durch wenigstens eine Kammer (15, 20, 27) bewirkt wird, wenn die genannte absorbierende Spitze (5) durch alle der genannten wenigstens einen Kammer (15, 20, 27) hindurchgeht.

3. Vorrichtung nach Anspruch 1, in welcher das genannte wenigstens eine Reagenz einen markierten Liganden oder einen markierten Rezeptor enthält.

4. Vorrichtung nach Anspruch 3, welche außerdem wenigstens eine weitere Kammer (15, 20, 27) und ein mikrobiologisches Wachstumsmedium in der genannten weiteren Kammer (15, 20, 27) enthält.

5. Vorrichtung nach Anspruch 3, welche außerdem wenigstens eine weitere Kammer (15, 20, 27) und ein Extraktionsreagenz zum Freisetzen antigener Determinanten oder verborgener Epitope eines Antigens in der genannten weiteren Kammer (15, 20, 27) enthält.

6. Vorrichtung nach Anspruch 3, in welcher das genannte markierte Ligand- oder markierte Rezeptorreagenz lyophylisiert ist.

7. Vorrichtung nach Anspruch 3, in welcher das genannte markierte Ligand- oder markierte Rezeptorreagenz ein Chromophor enthält.

8. Vorrichtung nach Anspruch 7, in welcher das genannte Chromophor ausgewählt ist aus der Gruppe bestehend aus Farbstoffen, gefärbten Partikeln, Pigmenten, Metallsolpartikeln oder farbstoffeingekapselten Liposomen.

9. Vorrichtung nach Anspruch 8, in welcher das genannte Chromophor ein Metallsolpartikel ist, ausgewählt aus der Gold, Silber oder die Kombination von Gold und Silber enthaltenen Gruppe.

10. Vorrichtung nach Anspruch 1, in welcher die genannte absorbierende Spitze (5) zum Sammeln einer Probe steril ist.

11. Vorrichtung nach Anspruch 3, welche außerdem wenigstens eine weitere Kammer (15, 20, 27) und Adsorptionspartikel in der genannten weiteren Kammer (15, 20, 27) enthält, wobei die genannten Adsorptionspartikel Bindemittel enthalten, zum Entfernen inhibierender oder Konkurrenzreaktionen auslösender Substanzen von der genannten Vorrichtung; und wenigstens eine weitere poröse Membran (25), und wobei
a) die genannten Adsorptionspartikel größer sind als die genannten markierten Liganden oder markierten Rezeptoren, und
b) die genannte wenigstens eine weitere poröse Membran (25) eine effektive Porengröße hat, die kleiner ist als die genannten Adsorptionspartikel und größer als die genannten markierten Liganden oder markierten Rezeptoren.

12. Vorrichtung nach Anspruch 1, in welcher die genannte wenigstens eine poröse Membran (18, 19) eine Vielzahl von porösen Membranen (18, 19) ist, von denen wenigstens eine Membran (19) mit verschiedenen Liganden oder Rezeptoren beschichtet ist.

13. Vorrichtung nach Anspruch 3, in welcher eine der genannten wenigstens einen Kammer (15, 20, 27) ligand- oder rezeptorbeschichtete Partikel enthält, welche bezüglich des durchschnittlichen Partikelgrößendurchmessers größer sind als der genannte markierte Ligand oder der genannte markierte Rezeptor und die effektive Porengröße der genannten wenigstens einen porösen Membran (18, 19).

14. Verfahren zur Durchführung eines Ligand-Rezeptor-Assays, unter Verwendung eines Mittels zum Probensammeln, das die Schritte umfaßt;
a) das Aufbringen einer Probe auf das genannte Mittel zum Probensammeln;
b) das in Kontakt bringen der genannten Probe auf dem genannten Mittel zum Probensammeln mit wenigstens einem markierten Reagenz, welches zur Durchführung des genannten Assays erforderlich ist;
c) das Vorsehen wenigstens einer porösen Membran (18, 19), wie es zur Durchführung des genannten Assays erforderlich ist;
d) das Hervorrufen von Diffusion einer Flüssigkeit von den Reaktanten auf das genannte Mittel zum Probensammeln nach dem genannten Schritt b) gleichzeitig auf alle der genannten wenigstens einen porösen Membran (18, 19); und
e) das Untersuchen aller der genannten wenigstens einen porösen Membran (18, 19) nach Durchführung aller obigen Schritte a) bis d); wobei jede Farbänderung oder dergleichen, die durch einen markierten Liganden oder einen markierten Rezeptor verursacht wird, der auf der genannten wenigstens einen Membran (18, 19) festgehalten oder nicht festgehalten wird, in Übereinstimmung mit dem Protokoll des genannten Essays schnell beobachtet werden kann.

15. Verfahren nach Anspruch 14 und die zusätzlichen Schritte des Bereitstellens zweier der genannten porösen Membranen (18, 19), was eine der genannten zwei porösen Membranen (18, 19) zu einer Kontrollmembran (18) und die andere der genannten zwei porösen Membranen (18, 19) zu einer Abfangmembran (19) macht, und das Anbringen der beiden genannten Membranen (18, 19) in der Weise, daß die genannten zwei Membranen (18, 19) gleichzeitig beobachtet werden können.

16. Verfahren nach Anspruch 14 und der zusätzliche Schritt der Filterung der genannten Reaktanten während des genannten Schritts d) durch Verwendung eines Filtermittels (25), welches eine Porengröße hat, die so ausgewählt ist, daß sie groß genug ist, um die genannte Diffusion der Flüssigkeit des genannten Schritts d) zu erlauben und klein genug ist, um Abriebteilchen, welche größer als die genannte ausgewählte Porengröße sind, und dergleichen abzuhalten, die durch den Kontakt mit einer der genannten wenigstens einen porösen Membran (18, 19) auf dem genannten Mittel zum Probensammeln sein können.

17. Verfahren nach Anspruch 14 und Durchführung des genannten Schrittes d) unter Einschluß eines Unterschritts, bei dem gleichzeitig wenigstens indirekt physikalischer Druck zwischen dem genannten Mittel zum Probensammeln und allen genannten wenigstens einer der porösen Membranen (18, 19) erzeugt wird.

18. Verfahren nach Anspruch 14, bei dem die genannten Schritte c) und d) unter Verwendung einer vorgefertigten Assay-Vorrichtung (33) ausgeführt werden und die Schritte zum Aufbau der genannten Assay-Vorrichtung (33) in Form eines Laminats, welches folgende aufeinanderfolgende Schichten enthält:
i) die genannte wenigstens eine poröse Membran (18,19);
ii) Mittel zur undurchlässigen Abschirmung (20) und die Schritte des Bereitstellens wenigstens einer Öffnung (21, 22) in dem genannten Mittel zur undurchlässigen Abschirmung (20) gleich der Anzahl der genannten wenigstens einen porösen Membran (18, 19), und das Bemessen und Anordnen der genannten wenigstens einen Öffnung (21, 22), so daß der genannte Schritt d) durch alle genannten wenigstens eine der Öffnungen (21, 22) gleichzeitig durchgeführt werden kann;
iii) Filtermittel (25) und das Auswählen einer Porengröße für das genannte Filtermittel (25), welche groß genug ist, um die Diffusion von Flüssigkeit nach dem genannten Schritt d) zu erlauben, und klein genug ist, um Abriebteilchen, die größer als die genannte ausgewählte Porengröße sind und dergleichen abzuhalten, welche sich durch Kontakt mit einer der genannten wenigstens einen der porösen Membranen (18, 19) auf dem Probensammler befinden können.

19. Verfahren nach Anspruch 18 und die zusätzlichen während des genannten Aufbaus der genannten geschichteten Vorrichtung durchgeführten Schritte zur Bereitstellung einer Schicht aus absorbierendem Mittel (17) und zum Anbringen der genannten Schicht aus absorbierendem Mittel (17) nächst der genannten wenigstens einen porösen Membran (18, 19) auf der der genannten undurchlässigen Abschirmung gegenüberliegenden Seite.

20. Vorgefertigte Assay-Vorrichtung (33) zur Verwendung mit einer Probensammelvorrichtung, welche aufeinanderfolgende Schichten umfaßt von:
a) wenigstens einer porösen Membran (18, 19);
b)
i) einem Mittel zur undurchlässigen Abschirmung (20), gestaltet mit wenigstens einer Öffnung (21, 22) in der Anzahl gleich der Anzahl der genannten wenigstens einen porösen Membran (18, 19);
ii) einem Mittel zum Bemessen und Anordnen der genannten wenigstens einen Öffnung (21, 22), so daß die Diffusion von Flüssigkeit zwischen der genannten wenigstens einen porösen Membran (18, 19) und einem Reaktanten, der in Kontakt mit der genannten Assay-Vorrichtung (33) gebracht wird, durch alle genannten wenigstens eine der Öffnungen (21, 22) gleichzeitig erfolgen kann; und
c) Filtermittel (25), wobei das genannte Filtermittel (25) ein Porengröße hat, die groß genug ist, um die genannte Diffusion von Flüssigkeit zu ermöglichen und klein genug ist, um Abriebteilchen, die größer als die genannte ausgewählte Porengröße sind, vom Kontakt mit jeder genannten wenigstens einen der porösen Membranen (18, 19) abzuhalten; und wobei die genannte vorgefertigte Assay-Vorrichtung (33) und die genannte Probensammelvorrichtung zusammen ein in sich geschlossenes Ligand-Rezeptor-Assay beinhalten.

21. Vorrichtung nach Anspruch 20 und eine Probensammelvorrichtung, die ein verlängertes Stäbchen (2) und eine absorbierende Spitze (5) umfaßt.

22. Vorrichtung nach Anspruch 21 und ein Behälter, der flüssiges markiertes Rezeptor- oder markiertes Ligand-Reagenz enthält.

23. Vorrichtung nach Anspruch 22, wobei das genannte markierte Reagenz ein Chromophor enthält.

24. Vorrichtung nach Anspruch 23, wobei das genannte Chromophor ausgewählt ist aus der Farbstoffe, gefärbte Partikel, Pigmente, Metalsolpartikel oder farbstoffeingekapselte Liposomen enthaltenden Gruppe.

25. Vorrichtung nach Anspruch 21 und eine Sandwichvorrichtung (37), wobei die genannte Sandwichvorrichtung (37) ein Plattenpaar (29, 30) und ein Mittel (32a) zum Zusammenklappen der genannten Platten (29, 30) längs einer jeweiligen Seite des genannten Plattenpaares (29, 30) enthält, und die genannte vorgefertigte Assay-Vorrichtung (33) zwischen den genannten Platten (29, 30) angebracht wird, und Mittel (34), um Druck auf die genannte Spitze (5) auszuüben, wenn die genannte Spitze (5) in Kontakt mit der genannten vorgefertigten Assay-Vorrichtung (33) ist.

26. Vorrichtung nach Anspruch 20 und die genannte Assay-Vorrichtung (33), welche eine absorbierende Schicht (17) in Kontakt mit der genannten wenigstens einen porösen Membran (18, 19) auf der der genannten undurchlässigen Abschirmung (20) gegenüberliegenden Seite einschließt.

27. Vorrichtung nach Anspruch 25 und die genannte Assay-Vorrichtung (33), welche eine absorbierende Schicht (17) in Kontakt mit der genannten wenigstens einen porösen Membran (18, 19) auf der der genannten undurchlässigen Abschirmung (20) gegenüberliegenden Seite einschließt.

28. Vorrichtung nach Anspruch 25, wobei das genannte Mittel zum Ausüben von Druck eine Klemmvorrichtung (34) umfaßt.

29. Vorrichtung nach einem der Ansprüche 25 bis 28, wobei die genannte Sandwich-Vorrichtung konturierte Vorsprünge (31, 32) enthält, die auf der genannten Sandwich-Vorrichtung (37) geformt sind, um Druck auszuüben.

## Revendications

1. Un dispositif à récepteur de ligands indépendant dont la combinaison comprend:-
(a) un dispositif de prélèvement ayant une longue tige (2) avec une pointe absorbante (5) pour prélever un échantillon;
(b) un tube (13) qui coopère avec le dispositif de prélèvement indiqué ci-dessus, le tube (13) ayant un espace intérieur, une extrémité ouverte et une extrémité opposée;
(c) au moins une chambre obturée hermétiquement (15, 20, 27) à l'intérieur du tube (13);
(d) plusieurs réactifs en nombre égal au moins au nombre de chambres (15, 20, 27) chaque réactif occupant une de ces chambres (15, 20, 27);
(e) un moyen pour obturer de manière étanche chaque chambre (15, 20, 27) contenant du réactif;
(f) le moyen d'obturation étanche étant constitué par des joints d'étanchéité pouvant être déchirés (7);
(g) un moyen à réaction entre récepteur et ligand comprenant au moins une membrane poreuse (18, 19);
(h) un trou (11) traversant la paroi, du tube (13) et situé à une distance prédéterminée par rapport à l'extrémité éloignée du tube (13);
(i) un moyen (20) pour fixer le moyen de réaction entre récepteur et ligand sur le tube (13) recouvrant le trou (11) avec au moins une membrane poreuse (12, 19) en contact avec l'espace intérieur du tube (13); et
(j) la longueur de la tige (2) ayant été étudiée pour permettre à la pointe absorbante (5) de traverser la ou les chambres (15, 20, 27) pour mélanger le ou les réactifs et pour permettre au fluide de se diffuser entre la pointe absorbante (5) et la ou les membranes (18, 19); qui permet
(k) d'immobiliser un ligand marqué ou un récepteur marqué sur le moyen de réaction entre récepteur et ligand (33a) et d'examiner le résultat de l'analyse.

2. Dispositif selon la revendication 1, un mécanisme de guidage pour le dispositif de prélèvement, comprenant en outre le moyen de guidage en question comprenant un guide et un moyen d'arrêt (3, 18′, 19′) un moyen à téton (4) le tube (13) et le guide et le moyen d'arrêt (3, 18′, 19′) ainsi que le téton (4) dont le fonctionnement conjoint provoque le déchirement successif du ou des joints déchirables (7) et faisant passer complètement la pointe absorbante (5) au travers de la ou des chambres (15, 20, 27) lorsque la pointe absorbante (5) traverse la ou toutes les chambres (15, 20, 27).

3. Dispositif selon la revendication 1, dans lequel le ou les réactifs contiennent un ligand marqué ou un récepteur marqué.

4. Dispositif selon la revendication 3, comprenant en outre au moins une autre chambre (15, 20, 27) et un milieu de croissance microbiologique dans cette autre chambre en question (15, 20, 27).

5. Dispositif selon la revendication 3 qui comprend en outre au moins une autre chambre (15, 20, 27) et un réactif d'extraction pour mettre à jour les déterminantes antigèniques ou les épitopes cachés d'un antigène dans l'autre chambre en question (15, 20, 27).

6. Dispositif selon la revendication 3, dans lequel le ligand marqué ou le réactif récepteur marqué sont liophylisés.

7. Dispositif selon la revendication 3, dans lequel le ligand marqué ou le réactif récepteur marqué sont constitués par un chromophore.

8. Dispositif selon la revendication 7, dans lequel le chromophore choisi peut être une teinture, des particules, des pigments, des particules métalliques en solution colloïdale ou des liposomes enrobés de teinture.

9. Dispositif selon la revendication 8, dans lequel le chromophore peut être une particule de solution colloïdale d'or, d'argent ou un mélange d'or et d'argent.

10. Dispositif selon la revendication 1, dans lequel la pointe absorbante (5) pour prélever des échantillons est stérile.

11. Dispositif selon la revendication 3, comprenant en outre au moins une autre chambre (15, 20, 27) et des particules d'adsorption dans l'autre chambre en question (15, 20, 27), les particules d'adsorption étant constituées par des liants pour supprimer les substances inhibitantes ou de réaction croisée produites par l'analyse; et au moins une autre membrane poreuse (25) et
(a) la dimension des particules d'adsorption est supérieure à celle des ligands marqués ou des récepteurs marqués, et
(b) au moins une membrane poreuse (25) ayant des pores de dimension effectivement plus faible que celle des particules d'adsorption et plus grande que celle des ligands marqués ou des récepteurs marqués.

12. Dispositif selon la revendication 1, dans lequel la membrane poreuse (18, 19) est en fait une pluralité de membranes poreuses (18, 19) dont au moins la membrane poreuse (19) est revêtue de différents ligands ou de différents récepteurs.

13. Dispositif selon la revendication 3, dans lequel la ou les chambres (15, 20, 27) contiennent des particules enrobées de ligand ou de récepteur et dont la dimension est supérieure au diamètre moyen de particule du ligand marqué ou du récepteur marqué dont il est question et supérieure à la dimension effective de la ou des membranes poreuses (18, 19).

14. Méthode d'analyse de récepteur de ligand utilisant un moyen de prélèvement d'échantillon, comprenant les phases suivantes:-
(a) mettre un échantillon sur le moyen de prélèvement d'échantillon;
(b) mettre en contact l'échantillon placé sur le moyen de prélèvement d'échantillon avec au moins un réactif marqué indispensable au déroulement de l'analyse;
(c) mettre au moins une membrane poreuse (18, 19) en fonction des nécessités de l'analyse à effectuer;
(d) faire diffuser le liquide des réactifs vers le moyen de prélèvement d'échantillon à la suite de l'étape (b) vers la ou toutes les membranes poreuses (18, 19) simultanément; et
(e) examiner la ou toutes les membranes poreuses (18, 19) après achèvement de toutes les phases (a) à (d) ci-dessus pour pouvoir observer facilement toute variation de couleur ou toute autre modification causée à un ligand marqué ou à un récepteur marqué capturé ou libre sur la ou les membranes (18, 19) selon la procédure de l'analyse effectuée.

15. Méthode selon la revendication 14, ainsi que les autres phases au cours desquelles on utilise deux membranes poreuses (18, 19) dont une est utilisée comme membrane poreuse de contrôle (18) et l'autre membrane comme membrane de capture (19), et on place les deux membranes (18, 19) pour qu'elles puissent être observées simultanément.

16. Méthode selon la revendication 14, avec une phase supplémentaire consistant à filtrer lesdits réactifs pendant le déroulement de la phase (d) à l'aide d'un moyen filtrant (25) dont la dimension de pore a été choisie pour qu'elle soit suffisamment grande pour permettre la diffusion du liquide au cours de la phase (d) et suffisamment faible pour empêcher tout contact entre les débris et autres éléments de dimension supérieure à la taille des pores choisie qui peuvent se trouver sur le moyen de prélèvement d'échantillon et une ou les membranes poreuses (18, 19).

17. Méthode selon la revendication 14, ainsi que l'exécution de la phase (d) y compris une sous-phase au cours de laquelle une pression physique est exercée au moins indirectement entre le moyen de prélèvement d'échantillon et la ou les membranes poreuses (18, 19) simultanément.

18. Méthode selon la revendication 14, dans laquelle les phases (c) et (d) sont effectuées à l'aide d'un dispositif d'analyse préfabriqué (33) ainsi que les phases de construction de ce dispositif d'analyse (33) sous la forme d'un laminé comprenant successivement les couches suivantes:-
(i) au moins une membrane poreuse (18, 19);
(ii) un moyen servant d'écran imperméable (20) et les phases permettant de créer une ou plusieurs ouvertures (21, 22) dans ledit moyen servant d'écran imperméable (20) et dont le nombre correspond à celui de la ou des membranes poreuses (18, 19) et de donner une dimension aux ouvertures en question (21, 22) et de les grouper de manière à ce que les opérations de la phase (d) puissent se dérouler simultanément au travers de la ou des ouvertures (21, 22); et
(iii) un moyen filtrant (25) et le choix d'une dimension de pore pour ce moyen filtrant (25) suffisamment grande pour permettre la diffusion du liquide de la phase (d) mais de dimension suffisamment petite pour empêcher les débris et autres éléments de dimension supérieure à celle choisie pour les pores déposés sur le moyen de prélèvement d'échantillons d'atteindre la ou les membranes poreuses (18, 19).

19. Méthode selon la revendication 18, ainsi que les phases supplémentaires qui se déroulent pendant la construction dudit dispositif laminé et ainsi que la phase consistant à placer une couche d'absorbant (17) à côté de la ou des membranes poreuses (18, 19) sur le côté de ces membranes opposé au moyen à écran imperméable (20).

20. Dispositif d'analyse préfabriqué (33) pour utilisation avec un dispositif de prélèvement d'échantillon comprenant successivement les couches suivantes:-
(a) au moins une membrane poreuse (18, 19);
(b)
(i) un moyen servant d'écran imperméable (20) qui comporte au moins une ouverture (21, 22) et dont le nombre est égal à celui de la ou des membranes poreuses (18, 19);
(ii) un moyen pour dimensionner et grouper au moins une ouverture (21, 22) de façon à ce que la diffusion du liquide entre au moins une membrane poreuse (18, 19) et un réactif amené en contact avec ledit dispositif d'analyse (33) puisse se produire simultanément au travers de la ou des ouvertures (21, 22); et
(c) un moyen filtrant (25) ayant des pores de dimension suffisamment grande pour permettre la diffusion du liquide et suffisamment petit pour empêcher que les débris et autres éléments de dimension plus grande que la dimension choisie pour les pores soient en contact avec la ou les membranes poreuses (18, 19), ce dispositif d'analyse préfabriqué (33) et le dispositif de prélèvement d'échantillon étant tous deux constitué par un dispositif à récepteur de ligand indépendant.

21. Dispositif selon la revendication 20, et le dispositif de prélèvement d'échantillon comprenant une longue tige (2) et une pointe absorbante (5).

22. Dispositif selon la revendication 21 et un récipient contenant un récepteur marqué liquide ou un réactif ligand marqué.

23. Dispositif selon la revendication 22, dans lequel le réactif marqué est un chromophore.

24. Dispositif selon la revendication 23, dans lequel le chromophorme choisi peut être soit une teinture, des particules teintes, des pigments, une solution colloïdale de particules métalliques ou des liposomes enrobés de teinture.

25. Dispositif selon la revendication 21, et un dispositif de type sandwich (37) constitué par une paire de moyens en forme de plaquettes (29, 30) un moyen (32a) servant de charnière entre les moyens en forme de plaquettes (29, 30) sur un des bords de ces plaquettes (29, 30), le dispositif d'analyse préfabriqué (33) étant placé entre les moyens en forme de plaquettes (29, 30) et un moyen pour exercer une pression sur la pointe absorbante (5) pour la maintenir en contact avec le dispositif d'analyse préfabriqué (33).

26. Dispositif selon la revendication 20, et le dispositif d'analyse (33) y compris une couche d'absorbant (17) en contact avec la ou les membranes poreuses (18, 19) sur le côté de la ou des membranes opposé à l'écran imperméable (20).

27. Dispositif selon la revendication 25 et le dispositif d'analyse (33) y compris une couche absorbante (17) en contact avec la ou les membranes poreuses (18, 19) sur le côté de la membrane opposé à l'écran imperméable.

28. Dispositif selon la revendication 25, dans lequel le moyen d'exercer une pression est un moyen de serrage (34).

29. Dispositif selon l'une quelconque des revendications 25 à 28, dans lequel le moyen à dispositif type sandwich utilisé pour exercer une pression comporte un moyen formé en creux comportant une partie en relief (31, 32) situé à l'intérieur du dispositif type sandwich (37).
